# FASCICULE DE BREVET EUROPEEN

(11) **EP 0 423 297 B1**
(45) Date de publication et mention de la délivrance du brevet: **21.09.1994**
(21) Numéro de dépôt: 90907120.1
(22) Date de dépôt: 25.04.1990
(51) Int. Cl.: A61F 2/08, A61F 2/40, A61F 2/02

(54) **DISPOSITIF DE RENFORT ET DE SOUTIEN DE LA COIFFE DES ROTATEURS D'UNE ARTICULATION D'EPAULE D'INDIVIDUS**
EINRICHTUNG ZUR VERSTÄRKUNG UND UNTERSTÜTZUNG EINER SCHULTERGELENKSKAPSEL
DEVICE FOR REINFORCING AND SUSTAINING THE CAP OF THE ROTATORS OF A HUMAN SHOULDER JOINT

(30) Priorité: 27.04.1989 FR 8906005
(43) Date de publication de la demande: 24.04.1991
(73) Titulaire: Gazielly, Dominique, F-42100 Saint Etienne (FR); BLONDEL, Pierre, 63600 Ambert (FR)
(72) Inventeur: Gazielly, Dominique, F-42100 Saint Etienne (FR); BLONDEL, Pierre, 63600 Ambert (FR)
(74) Mandataire: Dupuis, François
(86) Numéro de dépôt international: FR9000291
(87) Numéro de publication internationale: WO9012551

(56) Documents cités:
- EP-A- 0 169 045
- EP-A- 0 239 775
- DE-A- 3 008 270
- US-A- 4 347 847
- US-A- 4 769 038

## Description

L'invention se rattache au secteur technique de la chirurgie de l'épaule et des moyens, notamment pour en assurer la thérapie.

Afin de comprendre l'objet et l'intérêt de l'invention, il y a lieu préalablement d'exposer son environnement en regard des ruptures dégénératives de la coiffe des rotateurs.

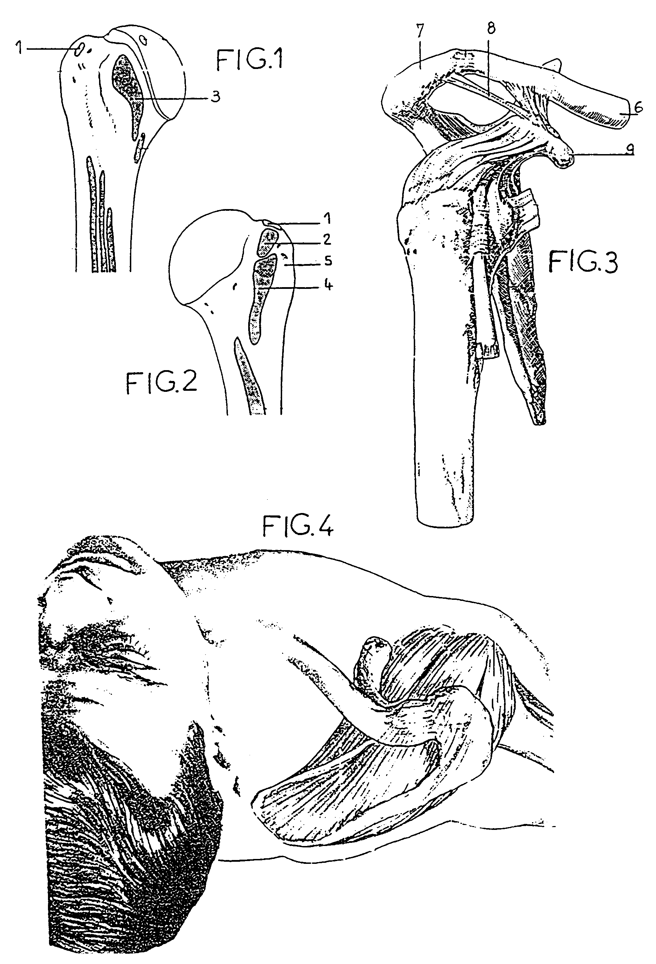

La coiffe des rotateurs d'une articulation d'épaule est constituée par la fusion de la portion tendineuse distale des quatre muscles sus et sous-épineux (1) (2), sous-scapulaire (3) et petit rond (4) (figures 1 et 2). Elle s'insère sur les faces supérieure. antérieure et postérieure du trochiter (5) en recouvrant ou plus précisément en coiffant le pôle supérieur de la tête humérale. D'une épaisseur de 3 à 4 millimètres. la puissance de ce manchon tendineux est en rapport avec le rôle fondamental de centreur et de stabilisateur de la tête humérale par rapport à la glisse lors des mouvements d'élévation antérieure et latérale et de rotation du bras.

La coiffe musculo-tendineuse passe sous une arche ostéo-fibreuse constituée d'avant en arrière par la portion de l'acromion (7) le ligament coraco-acromial (8) et l'apophyse coracoïde (9) (figure 3) en formant un canal. On a représenté par (6) la clavicule en coupe partielle. Une bourse de glissement s'interpose entre la coiffe musculotendineuse et les parois de l'arche ostéo-fibreuse. Il existe ainsi un conflit potentiel et réel entre la coiffe musculotendineuse et l'arche acromio- coracoïdienne, en particulier lors des mouvements d'élévation latérale et antérieure du bras. Le frottement répété de la coiffe contre les parois ostéo-fibreuses provoque une usure du manchon tendineux par laminage progressif. Le frottement peut être augmenté d'autant plus que les lésions d'arthrose avec ostéophytes agressifs peuvent épaissir les parois du canal précité, agressant encore plus la coiffe vieillie.

A terme, il se produit un amincissement progressif puis une perforation trophique (inférieure à 1 cm2) de la coiffe en particulier dans la région d'insertion du muscle sus-épineux, hypo-vascularisée et fragile. Une chute peut provoquer une rupture plus importante par désinsertion du muscle sus-épineux, avec extension vers l'avant (muscle sous-scapulaire) ou l'arrière (muscle sous-épineux). La rupture dégénérative de la coiffe des rotateurs ou musculo-tendineux peut être de taille variable :
- grade 1 - perforation (inférieure à 1 cm2) atteignant le muscle sus-épineux ;
- grade 2 - rupture du sus-épineux (supérieur à 1 cm2) ;
- grade 3 - La rupture est massive et concerne les muscles sus-épineux, sous-épineux, sous-scapulaire et parfois le petit rond.

La chirurgie de reconstruction de la coiffe des rotateurs est réalisable. Elle a pour but de recouvrir la tête humérale rendant à la coiffe son rôle de capteur et de stabilisateur et rétablissant un rythme scapulo-huméral harmo-nieux.

La reconstruction est obligatoirement associée à une excision du ligament coraco-acromial et à un nettoyage de l'espace sous-acromial avec notamment suppression des lésions d'arthrose et désépaississement de la portion antérieure de l' acromion.

Plusieurs procédés sont alors possibles pour recouvrir la tête humérale.

Certains procédés n'utilisent pas la coifie des rotateurs. La coiffe tendineuse a disparu par usure ou rétraction majeure. Il est techniquement possible de combler l'espace correspondant à la coiffe en recouvrant la tête humérale par un matériel inerte naturel ou synthétique. Il semble cependant préférable dans le cas de ruptures majeures où la tête humérale est décoiffée d'effectuer une plastie par lambeau musculaire deltoïdien antérieur, qui offre l'avantage de recouvrir la tête humérale et d'avoir un effet d'abaissement de la tête humérale par contraction active du lambeau.

D'autres procédés chirurgicaux utilisent la coiffe des rotateurs.

La coiffe des rotateurs est désinsérée. La tête humérale est décoiffée et le manque de couverture est plus ou moins important (grades 1, 2, 3). Les tendons sont rétractés suivant un degré variable, mais il est possible de libérer les adhérences de façon à les ramener vers leur zône d'insertion initiale trochitérienne. A la manière d'un toit mobile, la coiffe, rétractée vers l'omoplate, vient à nouveau recouvrir la tête humérale. Il reste donc à attacher les tendons sur le trochiter par des sutures au fil non résorbable, passées au fond d'une tranchée osseuse. La reconstruction doit être isométrique permettant sous contrôle visuel en peropératoire, des mouvements de rotation interne et externe coude au corps, et des mouvements de flexion et d'extension; sans lâchage des sutures de réinsertion transosseuse.

Si l'importance de la perte de substance tendineuse ou/et le degré de rétraction empêchent une réinsertion solide et fiable autorisant une rééducation immédiate et postopératoire, il est préférable d'avoir recours à un lambeau deltoïdien plutôt que d'essayer de combler l'espace laissé libre par la coiffe restant après une réinsertion aléatoire par un matériau synthétique ou autre. Certains auteurs préconisent en cas de rétraction moyenne ne permettant pas une réinsertion directe du tendon du muscle sus-épineux des lambeaux en rotation du muscle sous-scapulaire et/ou du muscle sous-épineux.

On connait par ailleurs par l'art antérieur le brevet EP-A-169045 qui définit une prothèse constituéé de différents types de brins et filaments et qui sont susceptibles de constituer un ligament ou tendon. Cette prothèse comprend un élément support autour duquel est attaché et fixé une pluralité de filaments fins ayant une grande capacité élastique - Cette prothése n'est cependant pas adapteé aux problèmes posés à la thérapie de la coiffe des rotateurs.

On connait egalement le brevet DE-A 3008270 qui décrit une prothese sous forme d'un bandeau avec une agrate de mise en tension, realisé en un matériau polyéthylène - ce bandeau n'est cependant pas applicable à la chirurgie de l'épaule et plus particulièrement au domaine de l'invention.

Selon les explications ci avant détaillées on comprend donc que la chirurgie de l'épaule est délicate, complexe et qu'un maximum de précautions doivent être prises pour en quelque sorte garantir ou fiabiliser l'intervention effectuée. On doit également tenir compte que les tissus sont usés, amincis et vieillis.

C'est pourquoi un premier but selon l'invention était de chercher à renforcer les tendons réinsérés, mais amincis ou fragilisés notamment par des infiltrations de corticoïdes et ainsi assurer une thérapie chirurgicale de la rupture ou de l'affaiblissement de la coiffe des rotateurs d'une articulation d'épaule d'un individu.

Un autre but selon l'invention était de réaliser un dispositif simple, de fabrication aisée et qui soit biologiquement compatible.

Un autre but selon l'invention était d'assurer un renforcement mécanique pur par addition de substance permettant de protéger la zône de réinsertion tendineuse et autorisant une rééducation post-opératoire plus active.

Un autre but selon l'invention était d'obtenir à terme, un épaississement des tendons, permettant un effet de renfort biologique définitif par intégration du matériau apporté initialement.

Ces buts et d'autres encore ressortiront bien de la suite de la description.

Selon l'invention, le dispositif utilisé pour la thérapie chirurgicale de la rupture ou affaiblissement de la coiffe des rotateurs d'une articulation d'épaule d'individu est caracterisé en ce que il est constitué par une bande stérile plate en matière biocompatible présentant un talon arrière à fixer sur le trochiter ou sur la masse tendineuse de la coiffe et se prolongeant linéairement ou par au moins deux branches divergentes vers le haut de la coiffe s'appliquant et se fixant sur le ou les tendons.

Ces caractéristiques et d'autres encore ressortiront bien de la suite de la description.

Pour fixer l'objet de l'invention illustrée d'une manière non limitative aux figures des dessins où :
Les figures 1 et 2 sont des vues partielles de la face antérieure et de la face postérieure de l'extrémité supérieure de l'humérus droit de l'individu.
La figure 3 est une vue latérale externe des articulations de l'épaule droite.
La figure 4 est une vue de dessus montrant la coiffe des rotateurs.
La figure 5 est une vue du dispositif de maintien et de soutien des tendons selon l'invention comprenant un talon et se prolongeant avec deux branches.
La figure 6 est une vue du dispositif en variante comprenant un talon et trois branches.
La figure 7 est une vue illustrant l'application du dispositif selon l'invention pour le soutien de certains tendons.

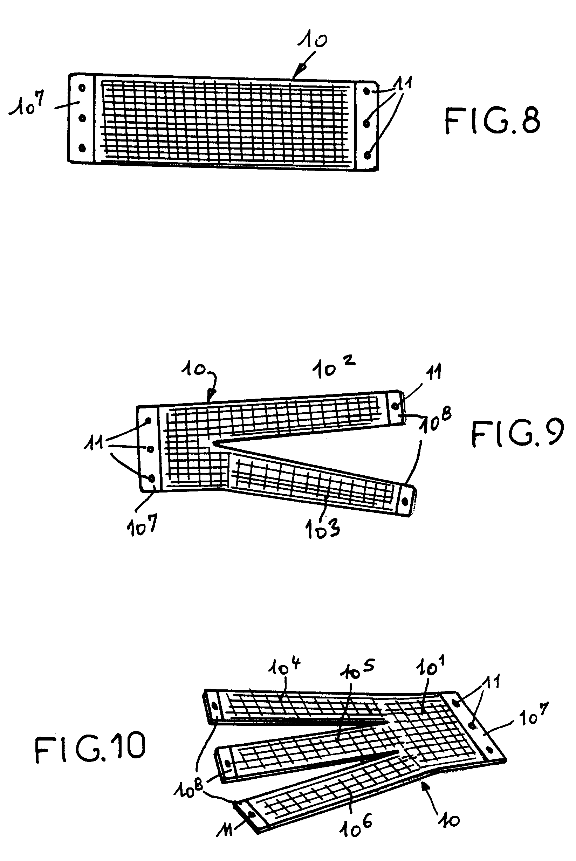

La figure 8 montre le dispositif vu en plan dans une forme géométrique simple.
Les figures 9 et 10 sont des vues montrant le dispositif des figures 5 et 6 aménagé à ses extrémités avec des moyens de fixation.

Afin de rendre plus concret l'objet de l'invention, on le décrit maintenant d'une manière non limitative en se référant aux exemples de réalisation des figures des dessins.

Le dispositif selon l'invention est utilisé dans la thérapie chirurgicale de la rupture ou de l'affaiblissement de la coiffe des rotateurs d'une articulation d'épaule.

Le dispositif se présente sous la forme d'une bande (10) de forme géométrique simple rectangulaire ou trapézoïdale, ou complexe à deux ou plusieurs branches (10.2, 10.3, 10.4, 10.5, 10.6). Le dispositif est réalisé selon une bande pouvant par exemple être tressée, tricotée, tissée, et plus généralement en tous matériaux biocompatibles. Cette bande est de faible épaisseur, stérile, biocompatible et courte. La bande présente d'une part une base ou talon (10.1) fixée par ses extrémités (10.7) sur le support trochitérien ou sur la masse tendineuse de la coiffe, et d'autre part se prolonge linéairement ou par deux ou trois branches divergeant vers le haut de la coiffe selon les axes de travail biomécaniques des tendons de la coiffe des rotateurs.

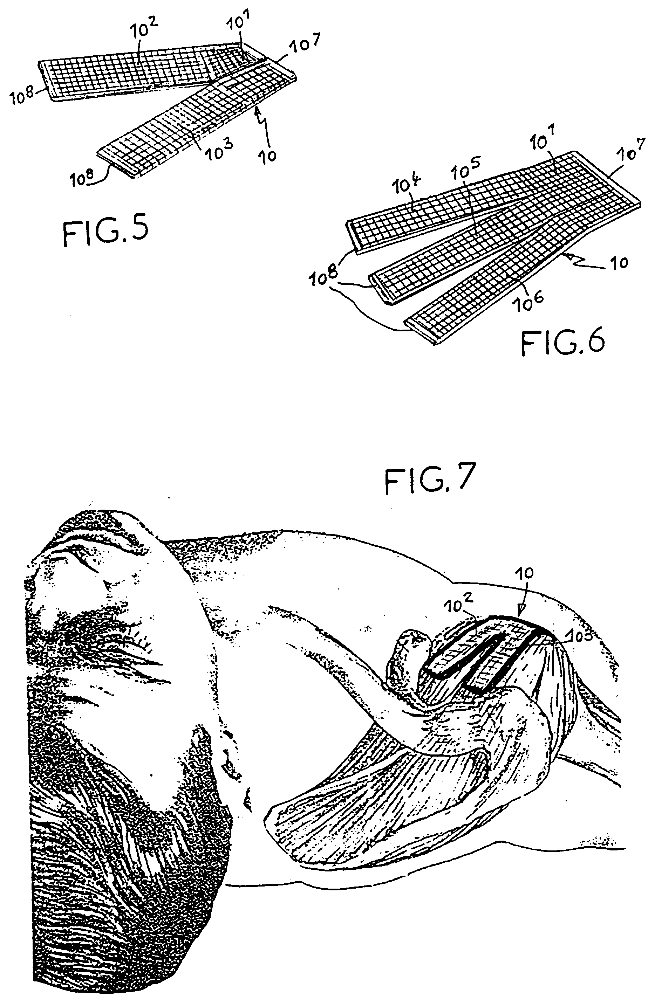

La bande se prolonge vers l'avant par au moins deux branches (10.2 - 10.3) divergentes définissant une forme en Y en étant susceptibles de s'appliquer et se fixer sur les tendons (figure 5). En variante, selon la figure 6, la bande présente trois branches (10.4, 10.5, 10.6) divergentes en forme de ψ. L'aspect général de la bande ainsi réalisée présente une forme rectangulaire ou trapézoïdale et permet à la coiffe renforcée de travailler suivant des lignes de forces biomécaniques, dans la direction des différents tendons. Selon un positionnement particulier, l'une des branches (10.2 - 10.3) ou (10.4, 10.5, 10.6) de la bande est fixée et suturée par son extrémité (10.8) sur le tendon réparé, aminci et ainsi renforcé en le protégeant, tandis que la ou les autres branches de la bande sont suturées à leurs extrémités (10.8) sur un ou des autres tendons sains. La conformation de la bande avec trois branches permet ainsi une suture sur les tendons du sus-épineux, du sous-épineux et du sous-scapulaire.

Les branches (10.2, 10.3, 10.4, 10.5, 10.6) peuvent être de même longueur ou de longueur différente.

Dans sa conformation linéaire, la bande permet un travail de soutien du tendon en cours d'affaiblissement avant rupture.

Selon une autre disposition, les extrémités (10.7 et 10.8) de la bande sont constituées d'une masse semi-rigide ou souple résultant de la fusion des fils constitutifs. Elles peuvent comporter des trous (11) réalisés dans cette masse facilitant la ligature du dispositif sur le trochiter ou la masse tendineuse et sur le ou les tendons sains. Ces trous sont en nombre adapté pour permettre la ligature et fixation.

Ainsi, la conformation avec dispositif de maintien et de soutien selon l'invention lui permet d'épouser parfaitement les tendons de la coiffe des rotateurs en prenant le minimum de place pour ne pas provoquer un conflit antérieur sous-acromial iatrogène par augmentation d'encombrement du contenu.

Par ailleurs, le positionnement du dispositif doit s'effectuer sur une coiffe reconstruite, de façon isométrique pour permettre un fonctionnement des tendons dans le sens des fibres et sans traction excessive.

Selon une autre disposition de l'invention, la bande de soutien et de maintien est réalisée en un matériau biologiquement compatible, autorisant ainsi son intégration biologique au sein des tissus vivants. Ce matériau est avantageusement un polypropylène tressé. Le matériau choisi autorise une intégration progressive de la bande de renfort et de maintien en assurant un épaississement de la coiffe reconstruite et donc un renforcement biologique.

Les avantages ressortent bien de l' invention, on souligne en particulier la simplicité de conception de la bande de renfort et de maintien, son adaptation aisée sur une coiffe reconstruite et insérée.

## Revendications

1. Dispositif utilisé pour la thérapie chirurgicale de la rupture ou affaiblissement de la coiffe des rotateurs d'une articulation d'épaule d'individu, caractérisé en ce que il est constitué par une bande stérile plate (10) en matière biocompatible présentant un talon (10.1) arrière à fixer sur le trochiter ou sur la masse tendineuse de la coiffe et se prolongeant linéairement ou par au moins deux branches divergentes vers le haut de la coiffe s'appliquant et se fixant sur le ou les tendons.

2. Dispositif selon l'une quelconque des revendications 1 et 2, caractérisé en ce que la bande (10) comprend deux branches (10.2 - 10.3) définissant une forme complexe en Y.

3. Dispositif selon l'une quelconque des revendications 1, caractérisé en ce que la bande (10) comprend trois branches (10.4, 10.5, 10.6), définissant une forme complexe en ψ.

4. Dispositif selon la revendication 1, caractérisé en ce que la bande est tressée ou tricotée ou tissée.

5. Dispositif selon la revendication 1, caractérisé en ce que la bande est réalisée en polypropylène tressé.

6. Dispositif selon l'une quelconque des revendications 1, 2, 3 et 4, caractérisé en ce que la bande présente une forme trapézoïdale.

7. Dispositif selon l'une quelconque des revendications 1, 2 et 3, caractérisé en ce que les extrémités (10.7 - 10.8) sont constituées d'une masse semi-rigide ou souple résultant de la fusion des fils constitutifs, et comportant dans ces masses des trous (11) facilitant la ligature du dispositif sur le trochiter, ou la masse tendineuse, et sur le ou les tendons sains.

## Claims

1. Device used for the surgical therapy of the rupture or weakening of the cap of the rotators of the human shoulder joint, characterised in that it consists of a flat sterile band (10) in biocompatible material with a heel (10.1) behind for fixation to the greater tuberosity of humerus or to the tendinous mass of the cap and extending linearly or by at least two divergent branches towards the top of the cap which are applied and fixed to the tendon(s).

2. Device as claimed in claim 1, characterised in that the band (10) has two branches (10.2-10.3) forming a complex Y-shaped form.

3. Device as claimed in claim 1, characterised in that the band (10) has three branches (10.4, 10.5, 10.6) forming a complex ψ-shaped form.

4. Device as claimed in claim 1, characterised in that the band is braided or knitted or woven.

5. Device as claimed in claim 1, characterised in that the band is made from braided polypropylene.

6. Device as claimed in any of claims 1, 2 and 3, characterised in that the band has a trapezoid shape.

7. Device as claimed in any of claims 1, 2 and 3, characterised in that the extremities (10.7-10.8) are made up of a semi-rigid or supple mass resulting from the fusion of the constitutive threads, and bearing in these masses holes (11) facilitating the ligature of the device to the greater tuberosity of humerus or to the tendinous mass and to the healthy tendon or tendons.

## Patentansprüche

1. Einrichtung für die chirurgische Therapie einer abgerissenen bzw. geschwächten Kapsel der Drehmuskeln eines menschlichen Schultergelenks, dadurch gekennzeichnet, daß sie aus einer flachen sterilen Binde aus biokompatiblem Material (10) besteht, die ein Fersenstück (10.1) aufweist, das am Tuberculum majus oder an der Sehnenmasse der Kapsel befestigt wird und sich linear oder mit mindestens zwei auseinanderlaufenden Zweigen zum oberen Teil der Kapsel hin fortsetzt, wobei sie an der bzw. den Sehnen ansetzt und befestigt wird.

2. Einrichtung nach Anspruch 1, dadurch gekennzeichnet, daß die Binde (10) zwei Zweige (10.2 - 10.3) besitzt, die eine komplexe Y-Form beschreiben.

3. Einrichtung nach Anspruch 1, dadurch gekennzeichnet, daß die Binde (10) drei Zweige (10.4, 10.5, 10.6) besitzt, die eine komplexe ψ-Form beschreiben.

4. Einrichtung nach Anspruch 1, dadurch gekennzeichnet, daß die Binde geflochten oder gestrickt oder gewebt ist.

5. Einrichtung nach Anspruch 1, dadurch gekennzeichnet, daß die Binde aus geflochtenem Polypropylen besteht.

6. Einrichtung nach einem der Ansprüche 1, 2 und 3, dadurch gekennzeichnet, daß die Binde eine Trapezform aufweist.

7. Einrichtung nach einem der Ansprüche 1, 2 und 3, dadurch gekennzeichnet, daß die Endstücke (10.7 - 10.8) aus einer halbstarren bzw. flexiblen Masse bestehen, die sich aus der Verschmelzung der konstitutiven Garne ergibt, und in dieser Masse Löcher (11) aufweisen, die die Ligatur der Einrichtung am Tuberculum majus bzw. der Sehnenmasse und an der bzw. den gesunden Sehnen erleichtern.
